# EUROPEAN PATENT SPECIFICATION

(11) **EP 3 559 212 B1**
(45) Date of publication and mention of the grant of the patent: **18.02.2026**
(21) Application number: 17832244.2
(22) Date of filing: 27.12.2017
(51) Int. Cl.: C12N 5/071, C12N 5/0786, G01N 33/50

(54) **SCREENABLE LIVER DISEASE MODELS AND METHODS**
ÜBERPRÜFBARE LEBERKRANKHEITSMODELLE UND VERFAHREN
MODÈLES DE MALADIES HÉPATIQUES POUVANT ÊTRE CRIBLÉES ET MÉTHODES ASSOCIÉES

(30) Priority: 23.12.2016 GB 201622149
(43) Date of publication of application: 30.10.2019
(73) Proprietor: Insphero AG, 8952 Schlieren (CH)
(72) Inventor: MESSNER, Simon, 8049 Zurich (CH); KELM, Jens M., 8610 Uster (CH); KOSTADINOVA-ANGELOVA, Radina, 8046 Zürich (CH); MORITZ, Wolfgang, 5454 Bellikon (CH)
(74) Representative: Michalski Hüttermann & Partner Patentanwälte mbB
(86) International application number: PCT/EP2017/084665
(87) International publication number: WO 2018/115533

(56) References cited:
- US-A1- 2014 274 802
- MESSNER S ET AL: "Multi-cell type human liver microtissues for hepatotoxicity testing", ARCHIVES OF TOXICOLOGY, SPRINGER-VERLAG, BERLIN, DE, vol. 87, no. 1, 11 November 2012 (2012-11-11), pages 209 - 213, XP035157583, ISSN: 1432-0738, DOI: 10.1007/S00204-012-0968-2
- CATHERINE C. BELL ET AL: "Characterization of primary human hepatocyte spheroids as a model system for drug-induced liver injury, liver function and disease", SCIENTIFIC REPORTS, vol. 6, no. 1, 4 May 2016 (2016-05-04), XP055451253, DOI: 10.1038/srep25187
- KOSTADINOVA RADINA ET AL: "A long-term three dimensional liver co-culture system for improved prediction of clinically relevant drug-induced hepatotoxicity", TOXICOLOGY AND APPLIED PHARMACOLOGY, ACADEMIC PRESS, AMSTERDAM, NL, vol. 268, no. 1, 23 January 2013 (2013-01-23), pages 1 - 16, XP028994281, ISSN: 0041-008X, DOI: 10.1016/J.TAAP.2013.01.012
- MASAKAZU INAMORI ET AL: "An Approach for Formation of Vascularized Liver Tissue by Endothelial Cell-Covered Hepatocyte Spheroid Integration", TISSUE ENGINEERING PART A, vol. 15, no. 8, 1 August 2009 (2009-08-01), pages 2029 - 2037, XP055451437, ISSN: 1937-3341, DOI: 10.1089/ten.tea.2008.0403
- M JOS? G?MEZ-LECH?N ET AL: "Competency of different cell models to predict human hepatotoxic drugs", EXPERT OPINION ON DRUG METABOLISM & TOXICOLOGY, vol. 10, no. 11, 9 October 2014 (2014-10-09), GB, pages 1553 - 1568, XP055348088, ISSN: 1742-5255, DOI: 10.1517/17425255.2014.967680

## Description

### Field of the invention

The present invention relates to the field of 3D tissue cultures and its applications in drug testing.

### Introduction

Liver fibrosis is a common outcome of virtually all chronic hepatic insults including viral hepatitis (i.e., hepatitis B and C), alcoholic or obesity-associated steatohepatitis (i.e., nonalcoholic steatohepatitis (NASH)), parasitic disease (i.e., schistosomiasis), metabolic disorders (i.e., Wilson's), hemochromatosis and other storage diseases, congenital abnormalities, autoimmune and chronic inflammatory conditions (i.e., sarcoidosis), and drug toxicity, among others (Puche, Saiman, & Friedman, 2013).

Liver Fibrosis is defined by the World Health Organisation as the presence of excess collagen due to new fiber formation. The fibers result from production of extracellular matrix (ECM) due to activation of quiescent hepatic stellate cells into myofibroblasts.

Liver fibrosis is currently mainly studied in animal experiments, but also isolated cell types of the liver are employed to study fibrosis *in-vitro. In-vivo* experimental models of liver fibrosis summarized in the review from Liedtke et al., 2013. Briefly, the most common animal model for liver fibrosis is the surgical bile-duct ligation. Other models employ genetic engineered animals, such as Mdr2 knockout mice or dominant negative TGF-βRII mice. Another type of animal models induces liver fibrosis through injection of chemicals (e.g. Carbon Tetrachloride, Thiocetamide, Dimethylnitrosamine) or through feeding (e.g. high-fat diet, choline deficient diet).

Currently employed *in-vitro* models for liver fibrosis typically use 2D-cultures of hepatic stellate cell lines (HSC, e.g., hTERT-HSC, LX2) or freshly isolated human or rodent HSC. The common limitation of these culture systems are that the HSC activate in culture into myofibroblasts, due to the interaction of HSC with the 2D culture dish. In addition, these models are just an incomplete representation of the liver's morphology and physiology, which limits their use in preclinical applications, e.g., assaying agents for their risk to cause fibrosis.

In recent years, the use of 3D spheroid cultures has become a more applicable format to obtain biologically relevant in vitro liver system for the study of liver disease (van Grunsven, 2017). However, current state of the art does not describe the reconstitution of liver organoids through the assembly of the four major liver cell types, i.e. hepatocytes, Kupffer cells, stellate cells and endothelial cells, and its use for the study of human liver disease, in particular but not exclusively the progression from healthy liver to NASH/fibrosis.

Bell, C.C. et al. (Scientific Reports, 2016, 6:25187; DOI: 10.1038/srep25187) discloses a scalable 3D primary human hepatocyte spheroid system in chemically-defined, serum-free conditions, and suggest it as an *in vitro* system to study liver function, liver diseases, drug targets and long-term drug-induced liver injury. Fugett Abu-Absi, S., et al. (Cytotechnology, 2004, 45, 125-140) disclose the co-culture of rat hepatocytes and the rat hepatic stellate cell line HSC-T6, forming heterospheroids.

### Summary of the invention

It is one object of the present invention to provide a liver disease model which has a superior physiological behavior.

It is one further object of the present invention to provide a liver disease model which is suitable for studying any of Steatosis, Inflammation, Fibrosis and Cirrhosis and respective therapeutic strategies.

It is one further object of the present invention to provide a liver disease model which is suitable for high throughput screening purposes.

These and further objects are achieved by the subject matter of the independent claims, while the dependent claims as well as the specification disclose further preferred embodiments.

In a first aspect, the invention relates to a method of providing a model platform for liver disease modelling. The method comprises preparing an artificial spheroidal microtissue comprising primary human hepatocytes, primary human hepatic stellate cells and primary human Kupffer cells, comprising co-culturing primary human hepatocytes, primary human Kupffer and primary human stellate cells in a 3D hanging drop system or a 3D low adherence well in maintenance medium for liver 3D culture without any of a growth factor, retinol and free fatty acid supplementation, for 5 to 7 days. such that the artificial spheroidal microtissue adopts a basal state. The stellate cells have a quiescent state. The method furthermore comprises subsequently incubating the microtissue for 1 to 14 days in the presence of a monounsaturated fatty acid (MUFA) and a saturated fatty acid (SFA) in a molar ratio of MUFA:SFA of ≥ 1, thereby inducing a steatotic state in the microtissue. The microtissue is grown and cultivated in a hanging drop system or a low adherence well.

In a second aspect, the invention relates to a method of preparing a spheroidal microtissue comprising at least hepatocytes, hepatic stellate cells, hepatic inflammatory cells, and further endothelial cells. The method comprises the steps of: (a) producing or providing a microtissue comprising primary human hepatocytes, human Kupffer cells and human hepatic stellate cells by co-culturing primary human hepatocytes, primary human Kupffer and primary human stellate cells in a 3D hanging drop system or a 3D low adherence well in maintenance medium for liver 3D culture without any of a growth factor, retinol and free fatty acid supplementation, for 5 to 7 days, such that the artificial spheroidal microtissue adopts a basal state, wherein the stellate cells have a quiescent state, (b) producing or providing a suspension comprising sinusoidal endothelial cells, (c) dispensing said suspension into wells or vessels comprising one or more microtissues of step (a), and (d) incubating said wells or vessels to let sinusoidal endothelial cells adhere to the microtissues.

In a third aspect, the invention relates to a method for testing compounds suspected of promoting, causing, or increasing the risk of NAFLD/NASH/Fibrosis. The method comprises co-culturing for 5 to 7 days primary human hepatocytes, primary human Kupffer and primary human hepatic stellate cells in a 3D hanging drop system or a 3D low adherence well in maintenance medium for liver 3D culture without any of a growth factor, retinol and free fatty acid supplementation, such that an artificial spheroidal microtissue is obtained that adopts a basal state. The stellate cells have a quiescent state. The method further comprises the steps of a) exposing the obtained microtissue to one or more test compounds suspected of promoting, causing, or increasing the risk of NAFLD/NASH/Fibrosis, and b) carrying out an endpoint analysis of at least one genetic, physiologic and/or morphologic parameter relevant to the liver pathology of NAFLD/NASH/Fibrosis. The microtissue is grown and cultivated in a hanging drop system or a low adherence well.

### Embodiments of the invention

Before the invention is described in detail, it must be noted that, as used in the specification and the appended claims, the singular forms "a," "an" and "the" include singular and/or plural referents unless the context clearly dictates otherwise. Further, in the claims, the word "comprising" does not exclude other elements or steps.

It is moreover to be understood that, in case parameter ranges are given which are delimited by numeric values, the ranges are deemed to include these limitation values.

The microtissue (MT) that is obtained by or within a method according to the invention, is an artificial spheroidal microtissue, comprising at least hepatocytes and hepatic stellate cells, and further at least one type of hepatic inflammatory cells..

As used herein, the term "hepatic inflammatory cells, refers to cells in the liver that mediate inflammation, or are activated by inflammatory cytokines. Such cells are, for example, invariant natural killer cells, T lymphocytes, polymorphonuclear cells, or CD8 lymphocytes or a mixture thereof such as Peripheral Blood Mononuclear Cells (PBMCs).

Microtissues mimicking hepatic organoids have been disclosed in Leite et al., 2016. The organoids described therein consist of (i) hepatocytes derived from HepaRG hepatic stem cells plus (ii) hepatic stellate cells (HSC). The authors describe the use of organoids comprising these two cell types in testing drug-induced liver fibrosis in vitro.

The authors claim that their hepatic organoid culture model is the first that can detect hepatocyte-dependent and compound induced HSC activation, thereby representing an important step forward towards in vitro compound testing for drug-induced liver fibrosis.

However, Leite et al. do not disclose the use of inflammatory cells in the organoids they describe.

In the mictotissue that is obtained in a method according to the invention, the hepatic inflammatory cells are Kupffer cells. The inventors have realized that Kupffer cells (also known as Kupffer-Browicz cells) play an important role in the transition of fatty liver towards steatohepatitis and eventually into liver fibrosis (Duarte et al., 2015). They have realized that the use thereof in a spheroidal microtissue can contribute to generate better organoids for use in liver fibrosis research.

Kupffer cells are specialized macrophages located in the liver, where they form part of the mononuclear phagocyte system. Kupffer cell activation is not only responsible for early ethanol-induced liver injury, common in chronic alcoholics, but also in non-alcoholic fatty liver disease (NAFLD) promoting an inflammatory response which contributes to the development of non-alcoholic steatohepatitis (NASH), which has been recognized as major cause for liver fibrosis. Kupffer cells hence play an important role in the progression of NAFLD towards NASH and liver fibrosis.

In order to further increase the quality of the artificial spheroidal microtissue with respect to its usability as a liver NASH/fibrosis model, the inventors therefore suggest, for the first time, to incorporate also Kupffer cells into such model.

One other type of liver microtissue is provided by Organovo, and called "exVive 3D Liver". These tissue models are created using 3D bioprinting technology and different liver cell types. The resulting tissues contain a reproducible architecture and are being used for liver tissue-specific toxicity marker assessment as a supplement to in vitro and preclinical (non-GLP) animal testing.

For bioprinting these microtissues, bioinks are being prepared which comprise the cells that later form the tissues. The bioinks are then deposited in a two-compartment planar geometry onto the membranes of standard 24-well plates via continuous deposition microextrusion with non-parenchymal cells comprising the border regions of each compartment and the parenchymal cells filling each compartment (see Nguyen et al, 2016).

The tissues obtained by said 3D bioprinting process do not have a spheroidal shape - they rather come in cylindric shapes, and do hence have disadvantages as regards their physiological behavior.

The spheroidal shape cannot be produced with 3D bioprinting processes, but largely relies on self assembly of the cells, as will be described below. The spheroidal shape makes sure that cells receive enough oxygen and nutrients since the tissue diameter is below 500 µm. The small size of the spheroids needs only small amounts of the precious primary cell material and thus allows production of many spheroids from a limited amount of cellular material.

According to one embodiment of the method of the invention, the microtissue further comprises endothelial cells.

The endothelial cells act as a selective permeability barrier, in such way that they control which molecules enter, from the blood vessels, into the surrounding tissue including the hepatocytes.

Endothelial cells facilitate access of the hepatocyte to oxygen and small molecules in the microcirculation. Other specialized functions include clearance of colloids and macromolecules, promotion of hepatic stellate cell quiescence, and induction of immune tolerance.

The hepatic endothelial cell may be injured by a variety of toxins, ischemia-reperfusion, and even bacteria, leading to vascular liver diseases such as fibrosis.

Preferably, the endothelial cells are sinusoidal endothelial cells. Liver sinusoids are a type of sinusoidal blood vessel with fenestrated, discontinuous endothelium that serve as a location for mixing of the oxygen-rich blood from the hepatic artery and the nutrient-rich blood from the portal vein. Hepatocytes are separated from the sinusoids by the space of Disse.

Furthermore, the inventors have realized that sinusoidal endothelial cell keep quiescent in vivo, hence avoiding that they activate into myofibroblast. Hence, sinusoidal endothelial cell increase cellular life span within the microtissues and thus treatment time.

Sinusoidal endothelial cells play thus an important role in liver physiology and, as such, in liver fibrosis pathogenesis. In order to increase the quality of the artificial spheroidal microtissue with respect to its usability as a liver fibrosis model, the inventors therefore suggest, for the first time, to incorporate also sinusoidal endothelial cells into such model.

The incorporation of sinusoidal endothelial cells into spheroidal microtissues that comprise hepatocytes and stellate cells provides many technical challenges.

Further, it is a challenge to ensure that the liver sinusoidal endothelial cells (LEC) grow on the outer face of the spheroidal microtissues. In a mixed culture. LEC grow interspersed with the remaining liver cells, which would not sufficiently mimic the microanatomy of liver tissue. The inventors have hence developed a suitable coating protocol which makes sure that the LEC grow on the outside of the spheroidal microtissues, and hence can develop their function as endothelial cells.

According to one further embodiment of the method of the invention, the microtissue has a diameter of between ≥ 30 µm and ≤ 500 µm. Preferably, the diameter is ≤ 450 µm, more preferably ≤ 400 µm, even more preferably ≤ 350 µm and most preferably ≤ 300 µm.

According to one further embodiment of the method of the invention, the microtissue comprises between ≥ 500 and ≤ 10000 cells in total. Preferably, the microtissue comprises ≤ 8000 cells, more preferably ≤ 6000 cells, even more preferably ≤ 5000 cells and most preferably ≤ 3000 cells.

The exVive 3D Liver tissues discussed above have, next to their cylindrical shape, diameters of about 2.5 mm, and a thickness of about 0.4 mm (Organovo white paper of 2015), which makes them unsuitable for use in High Throughput Screening (HTS) environments, which usually require microtiter plates with 384 wells or more (see the plate types marked with an asterisk in the following table).

| Type of microtiter **plate** | Well diameter (bottom, mm) |
|---|---|
| 24 | 16 |
| 96 | 6.4 |
| 384* | 3.0 |
| 1536* | 1.6 |
| 3456* | < 1 |

Both the shape and the minimum size of these tissues are defined by the bioprinting technique.

In contrast thereto, the spheroidal microtissues obtained in a method according to the invention can be made with diameters of between ≥ 30 µm and ≤ 500 µm. Besides other advantages, including better oxygen transfer and better transparency for microscopical or video imaging applications, this size range allows their use in microtiter plates with 384, 1536 or even 3456 wells, making them suitable for HTS applications as well.

Furthermore, the exVive 3D Liver tissues comprise up to 10⁶ cells in total, which corresponds to approx. 300-400 times the cell number required for one liver microtissue of a size of 250 µm in diameter. Current bioprinting formats are much more demanding regarding to cell sourcing and associated costs. In this context it is important to understand that the provision of sufficient cell mass is a critical issue in primary cell culture, in particular when spheroidal 3D microtissues are made. This scrutiny is even fostered by the fact that in many cases, the cells used for spheroidal 3D microtissues are primary human cells that are derived from organ donations. Generally, organs are used which do not qualify for therapeutic transplantation. However, even these organs are in extremely short supply.

The spheroidal microtissues obtained in a method according to the invention have lower demands as regards resources. In fact, roughly two order of magnitude less cells are being required for microtissue formation. This means that more microtissues can be made from less donated livers.

According to one embodiment of the method of the invention, the sinusoidal endothelial cells form a cell layer around the three-dimensional microtissue comprising at least the hepatocytes and the hepatic stellate cells.

In this embodiment, the sinusoidal endothelial cells can efficiently act as a selective permeability barrier, in such way that they control which molecules enter, from the medium, into the artificial spheroidal microtissue including the hepatocytes, the stellate cells and, optionally, the Kupffer cells. Preferably, the sinusoidal endothelial cells form a monolayer on the microtissue surface.

According to one embodiment of the method of the invention, the cells in the microtissue are mammalian cells, preferably selected from the group consisting of
- human cells
- monkey cells
- pig cells
- canine cells, and/or
- rodent cells.

Intermediately, a) the microtissue comprises pro-activated stellate cells.

As obtained by a method according to the invention, b) the microtissue represents a liver model at basal state.

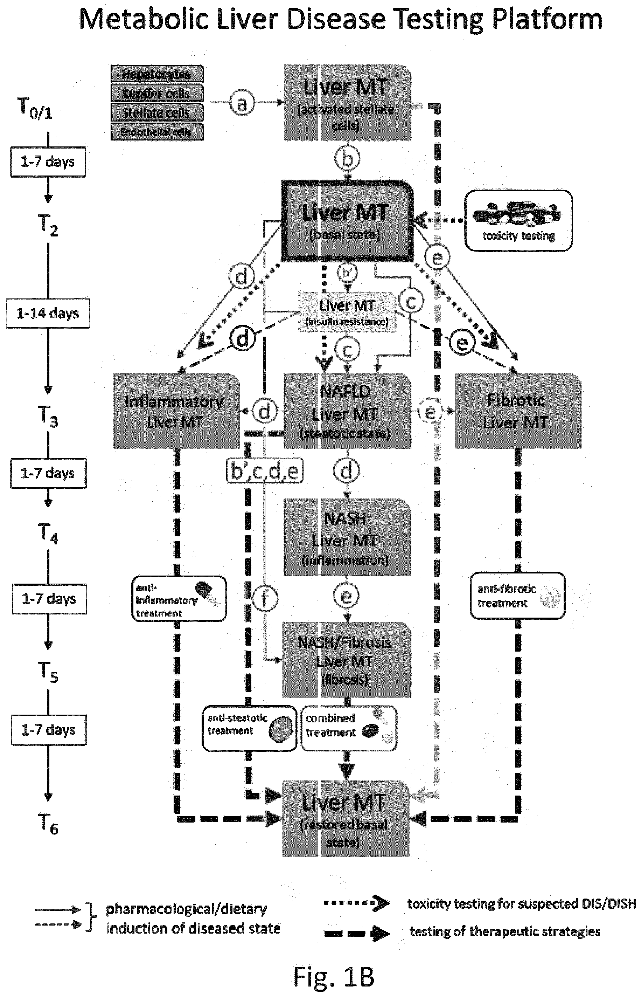

As regards option a) such microtissue is formed, e.g., at time T1, i.e., right after tissue formation. See process [a] as shown in Fig. 1B.

As regards option b) such microtissue can be obtained, e.g., after reversion from an activated post-production state. See process [b] as shown in Fig. 1B, and as e.g. described in example 8.

A microtissue obtained at basal state can be progressed to one of the states selected from the list consisting of:
a) insulin resistant state,
b) steatotic state,
c) inflammatory state,
d) fibrotic state,
e) NASH/Fibrosis phenotype, option 1,
f) NASH/Fibrosis phenotype, option 2.

As regards option a), this state can be induced, e.g., by, process [b'], e.g., by exposure to
- high glucose/fructose
- high insulin
- high free fatty acids (SFA>MUFA>PUFA)
- adipocyte derived cytokines TNF-α, IL-1β, and IL-6
- resistin
- glucocorticoids
- or a combination of above.

As regards option b), such state is induced in a method of the invention, by process [c] as shown in Fig. 1B, and e.g. described in example 3, with. e.g., a MUFA:SFA molar ratio ≥1.

As regards option c), such state can be induced, e.g., by process [d] as shown in Fig. 1B, and as e.g. described in example 4.

As regards option d), such state can be induced, e.g., by process [e] as shown in Fig. 1B, and as e.g. described in example 4 and 5.

As regards option e), such state can be induced, e.g., by sequential or simultaneous application of process [b'], [c], [d], and [e] as shown in Fig. 1B.

As regards option f), such state can be induced, e.g., by treatment with pro-inflammatory dietary free fatty acid composition (see, e.g., process [f] as shown in Fig. 1B.), with e.g. SFA only or MUFA:SFA molar ratio < 1.

The term "PUFA"; as used herein, means polyunsaturated fatty acids or fats. The term "MUFA"; as used herein, means monounsaturated fatty acids or fats. The term "SFA"; as used herein, means saturated fatty acids or fats.

Most common SFAs in human diet are: Stearic acid, Palmitic acid, Myristic acid, Lauric acid, Capric acid, Caprylic acid, Caproic acid. Most common MUFAs in human diet are: Oleic acid, Palmitoleic acid.

Also disclosed herein is a method of preparing a spheroidal microtissue comprising at least hepatocytes, hepatic stellate cells, hepatic inflammatory cells, and endothelial cells, which method comprises the steps of:
a) producing or providing a microtissue comprising at least hepatocytes, hepatic stellate cells and hepatic inflammatory cells,
b) producing or providing a suspension comprising sinusoidal endothelial cells,
c) dispensing said suspension into wells or vessels comprising one or more microtissues of step a), and
d) incubating said wells or vessels to let sinusoidal endothelial cells adhere to the microtissues.

In said process, the endothelial cells sink down into the wells or vessels comprising the microtissues after dispension or pipetting, and get in contact with the microtissues, where they adhere to the extracellular matrix and from cell-to-cell contacts.

In one example, the endothelial cells are sinusoidal endothelial cells.

According to one embodiment of the method of the invention, the microtissues are grown and cultivated in a hanging drop system.

Such hanging drop system is for example provided by InSphero's GRavityPLUS^{™} Hanging Drop System (CH), described in WO2010031194A. Methods of making microtissues in such hanging drop system are e.g. disclosed in WO2015158777A1.

According to one further embodiment of the method of the invention, the microtissues are grown and cultivated in a low adherence well.

Such low adherence well system is for example InSphero's GravityTRAP^{™} ULA Plate (CH). As an alternative, Microtiter plates treated with a cell repellent as for example provided by Greiner can be used. Methods of making microtissues in such system are e.g. disclosed in PCT/EP2016/065571.

At least some of the cells may be cryopreserved cells which are thawed before processing, i.e., before producing the microtissues. At least some of the cells may also be non-frozen cells.

According to one further example, a spheroidal microtissue is disclosed which has been obtained with a method according to the above description.

Such microtissue comprises at least hepatocytes, hepatic stellate cells and hepatic inflammatory cells (plus optionally an outer layer comprising sinusoidal endothelial cells).

It may have a diameter of between ≥ 30 µm and ≤ 500 µm. Preferably, the diameter is ≤ 450 µm, more preferably ≤ 400 µm, even more preferably ≤ 350 µm and most preferably ≤ 300 µm.

It may comprise between ≥ 500 and ≤ 10000 cells in total. Preferably, it comprises ≤ 8000 cells, more preferably ≤ 6000 cells, even more preferably ≤ 5000 cells and most preferably ≤ 3000 cells.

According to the invention, a method of providing a model platform for liver disease modelling is provided.

Such platform is capable to allow following investigative modes:
1. Study of disease progression and testing of anti-NASH/Fibrosis therapeutic or preventive strategies by pharmacological and/or dietary interventions, starting from different entry points.
2. Safety assessment of drug candidates with suspected liver toxic liabilities

In one example, the model platform allows at least one selected from the group consisting of:
- mechanistic studies of disease progression,
- prevention of disease progression by pharmacological or dietary intervention, and/or
- the reversion of diseased states.

In another example, the model platform is provided in an assay-ready format composed of basic media and reagents enabling the installment and recapitulation of disease specific states, representative of specific preventive and therapeutic entry points.

In one example, the platform further comprises quality-control data and calibration data, which data are specific to the production batch, and provide information about at least one feature selected from the group consisting of:
- Biomarkers for disease progression,
- Cell viability and functionality, and/or
- Microtissue size.

In still another example of the model platform, the liver disease is at least one selected from the group consisting of:
- liver fibrosis,
- viral hepatitis (e.g., hepatitis B and C),
- alcoholic or obesity-associated steatohepatitis (e.g., nonalcoholic steatohepatitis (NASH)),
- parasitic disease (e.g., schistosomiasis),
- metabolic disorders (e.g., Wilson's),
- hemochromatosis and other storage diseases,
- congenital abnormality,
- autoimmune and chronic inflammatory conditions (e.g., sarcoidosis), and/or
- drug toxicity.

In yet another example of the model platform, the spheroidal microtissue is in co-culture with at least one type of inflammatory cells.

In specific examples, such inflammatory cells are selected from the group consisting of:
- NK, NKT or iNKT cells,
- T lymphocytes,
- polymorphonuclear cells,
- CD8 lymphocytes, and/or
- Peripheral Blood Mononuclear Cells (PBMCs).

In one example, at least one type of inflammatory cells has been haplotype-matched to liver cells of the microtissue.

In one further example the microtissue and at least one type of inflammatory cells are
- in a static co-culture, or
- in a microfluidic device allowing free floating of inflammatory cells yet immobilized liver microtissues

The versatility of this platform allows different modes of implementation or commercialization:
1. Provision of aforementioned basal liver models and disease models as assay ready system, including
   - Basal maintenance medium
   - Diseased state-specific media
   - QC certification
   - Reference test compounds
2. Provision of aforementioned basal liver models and supplements for the induction of any of above mentioned disease states as assay-ready system, including
   - Basal maintenance medium
   - QC certification
   - Reference test compounds

Also disclosed herein is a liver model which has been obtained with a method according to any to the above description and/or comprises a spheroidal microtissue according to the above description and/or comprises a platform according to the above description.

According to one further aspect of the invention, an assay method for testing compounds suspected for promoting, causing, or increasing the risk of NAFLD/NASH/Fibrosis, is provided, which assay method comprises the steps of providing a spheroidal microtissue according to the invention and
a) treatment of one or more test microtissues with one or more test compounds suspected for promoting, causing, or increasing the risk of, NAFLD/NASH/Fibrosis, and
b) carrying out an endpoint analysis of at least one genetic, physiologic and/or morphologic parameter relevant to the respective liver pathology.

In a method according to the invention, said liver fibrosis is NAFLD or NASH/fibrosis. In specific embodiments, the said compounds suspected for promoting, causing, or increasing the risk of liver fibrosis are suspected to induce steatosis (DIS: drug-induced steatosis) or steatohepatitis (DISH: drug-induced steatohepatitis)

According to one further embodiment of said method of the invention, a positive control is furthermore used, which positive control comprises:
a') treatment of a control microtissue with a control agent selected from a group of fibrosis inducers (e.g. TGF-β1, methotrexate (MTX) or thioacetamide (TAA), steatosis inducers (e.g. cholesterol, fatty acids, amiodarone, tetracyclin, valproic acid) and/or inflammation inducers (e.g. TNF-α, Lipopolysaccharide, MTX)
b') carrying out the same endpoint analysis of at least one genetic, physiologic and/morphologic parameter, and

According to one further embodiment of said method of the invention the endpoint analysis refers to at least one parameter selected from the group consisting of:
- Release of one or more biomarkers, as for example measured by specific ELISAs, biochemical assays or by omics technologies, e.g. metabolomics, lipidomics, glycomics, and secretomics
- Lipid content of the microtissue cells
- Histology of the microtissue and its cells
- RNA expression marker such as alpha-smooth muscle actin, COL1A1, Vimentin or TIMP-1
- Size or volume of the microtissue, and/or
- Mitochondrial activity

Example classes of biomarkers for fibrosis include, inter alia, collagen release, fibronectin release, matrix metalloproteinase (MMP) release, tissue inhibitor of matrix metalloproteinase (TIMP) release, cytokine release.

Lipid content of the microtissue cells can be determined by Nile-red or Bodipy (boron-dipyrromethene) staining of triglycerides in microtissues and quantification, or Triglyceride quantification based on biochemical assays.

Histology of the microtissue and its cells can be determined by determination or measurement of ECM-proteins(Collagen 1A1, Collagen 3A1, Collagen 4A1, Laminin, Elastin, Fibronectin, CD44), Stellate cell markers (alpha-SMA, PDGFR-β, Vimentin, Desmin, GFAP), Kupffer cell markers (CD68, CD163), Hepatocyte markers (Albumin, CYP3A4), LEC-markers (CD31) or Steatosis markers (H&E staining; appearance of unstained, white droplets of small to large size in cytoplasma of hepatocytes or Oilred O stained cryosections).

A spheroidal microtissue according to the above description, or obtained with a method according to the above description, and/or comprising a platform according to the above description can be used for at least one purpose selected from the group consisting of:
- drug efficacy and/or toxicity screenings
- investigative/mechanistic toxicology,
- target discovery/identification,
- drug repositioning studies, and/or
- pharmacokinetics and pharmacodynamics assays.
is provided.

### Experiments and Figures

In the claims, the word "comprising" does not exclude other elements or steps, and the indefinite article "a" or "an" does not exclude a plurality.

### Figures

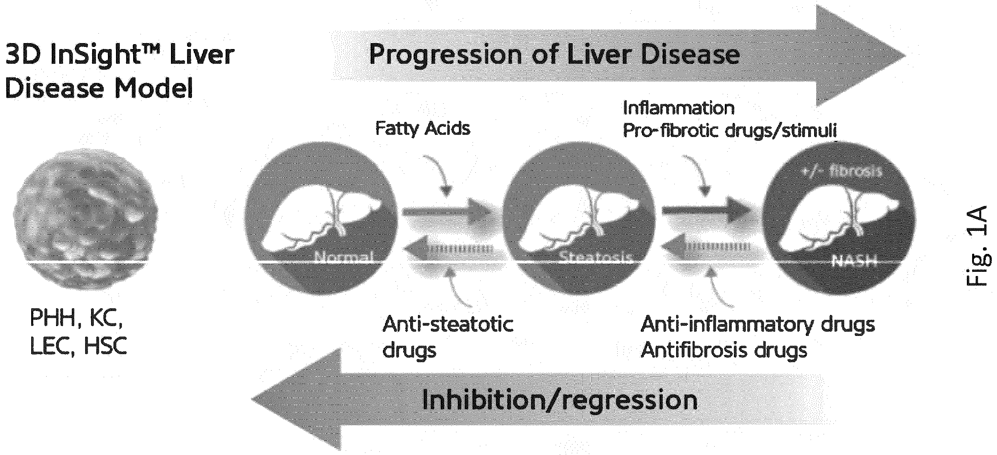

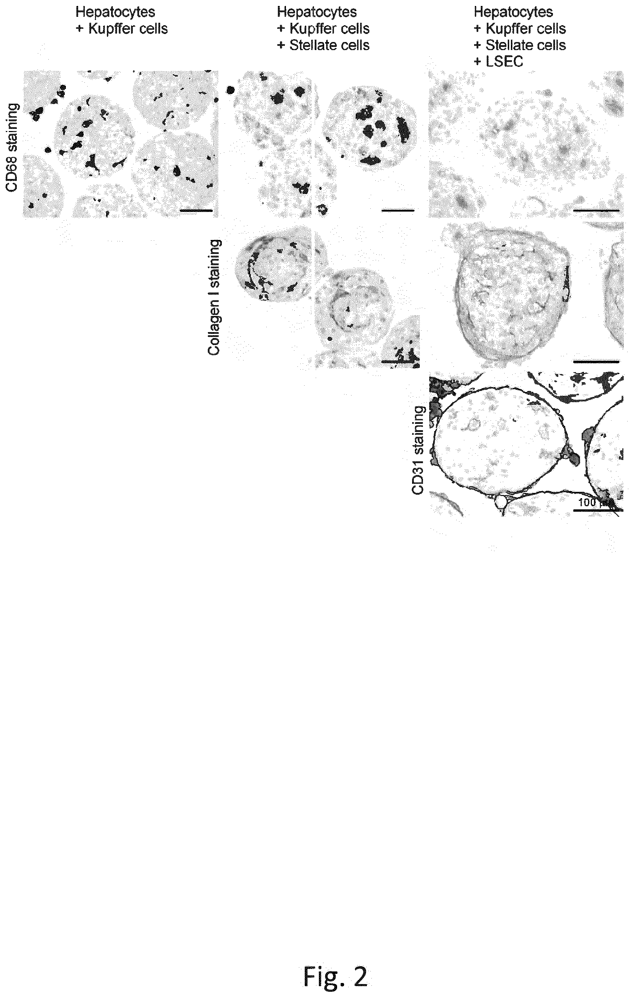

Fig. 1A: Mechanism of development of fibrosing non-alcoholic steatohepatitis (NASH). Key stimuli: accumulation of lipids (steatosis) and inflammation (triggered by activation of Kupffer cells). Key cell types: primary human hepatocytes (PHH), Kupffer cells (KC), Liver endothelial cells (LEC) and hepatic stellate cells (HSC). NASH is the intricate interaction of hepatocytes, Kupffer cells and stellate cells and long-term maintenance of signaling pathways leading to fibrosis.
Fig. 1B: Platform, in which the different liver states can be induced as described. The following sub-processes are provided:
   o Liver model with pro-activated stellate cells (T₁, right after tissue formation, process [a])
   o Liver model at basal state, after reversion from activated post-production state (process [b], described in example 8), or further progressed to either of the following states:
      ▪ Liver model with insulin resistant state (process [b']) introduced by
         - High glucose/fructose
         - High insulin
         - High free fatty acids (SFA>MUFA>PUFA)
         - Adipocyte derived cytokines TNF-α, IL-1β, and IL-6
         - Resistin
         - Glucocorticoids
         - or a combination of above
      ▪ Liver model with steatotic state, (process [c], described in example 3). • MUFA:SFA molar ratio ≥1
      ▪ Liver model with inflammatory state, (process [d], described in example 4).
      ▪ Liver model with fibrotic state, (process [e], described in example 4 and 5).
      ▪ Liver model with NASH/Fibrosis phenotype, (by sequential or simultaneous application of process [b'], [c], [d], and [e]).
      ▪ Liver model with NASH/Fibrosis phenotype, by treatment with pro-inflammatory dietary free fatty acid composition (process [f])
         - SFA only
         - or MUFA:SFA molar ratio <1
Fig. 2: Presence of the Hepatocytes, Kupffer cells, stellate cells and LEC's in human liver spheroids. Fig. 2 shows photomicrographs of microtissues made with the method according to the present invention, in a hanging drop system. CD68 is a glycoprotein which binds to low density lipoprotein, and is expressed, inter alia, on Kupffer cells. Collagen I is a marker for hepatic stellate cells, while CD31 is a protein that in humans is encoded by the PECAM1 gene, and makes up a large portion of endothelial cell intercellular junctions, hence a marker for endothelial cells. These cells have been coated to the microtissues in a two-step production process to form an endothelial layer between liver tissue and medium.

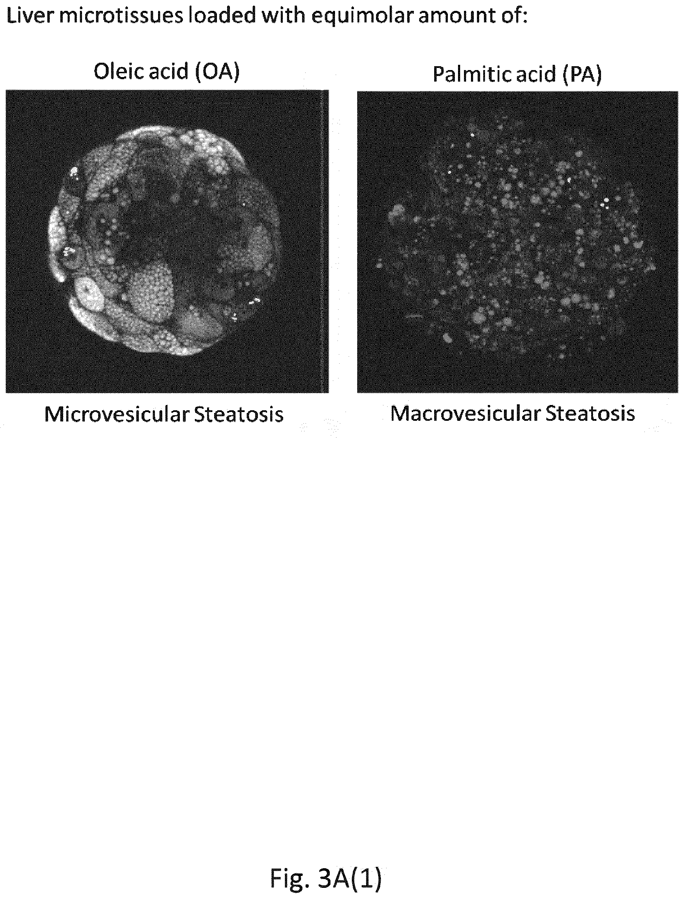

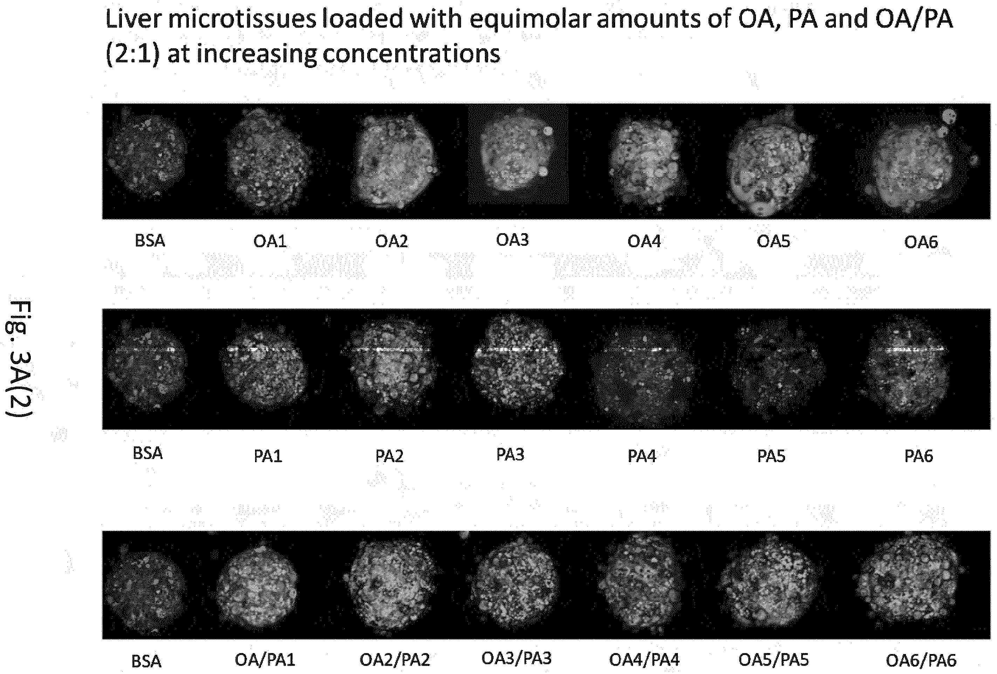

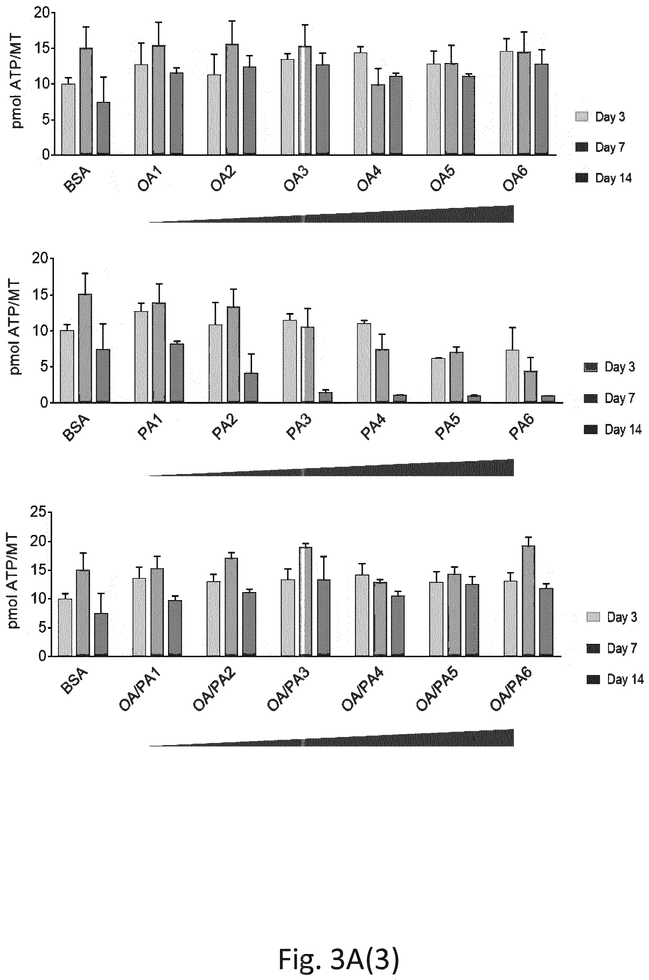

Fig. 3A(1): Induction of liver-disease phenotype "steatosis" as determined by lipid accumulation. Exposure to free fatty acids (Oleate and Palmitate) induced lipid accumulation in microtissues, visualized by nile-red staining and subsequent confocal fluorescence imaging. Images are depicted as maximal intensity projections.
Fig. 3A (2): Lipid accumulation in dependency of increasing concentrations of PA, OA or a combination thereof in a molar ratio of 2:1. The total amount of FFA was identical among the groups at a given concentration.
Fig. 3A(3): ATP measurements from identical groups after exposure to increasing concentrations of FFA for 3-14 days.

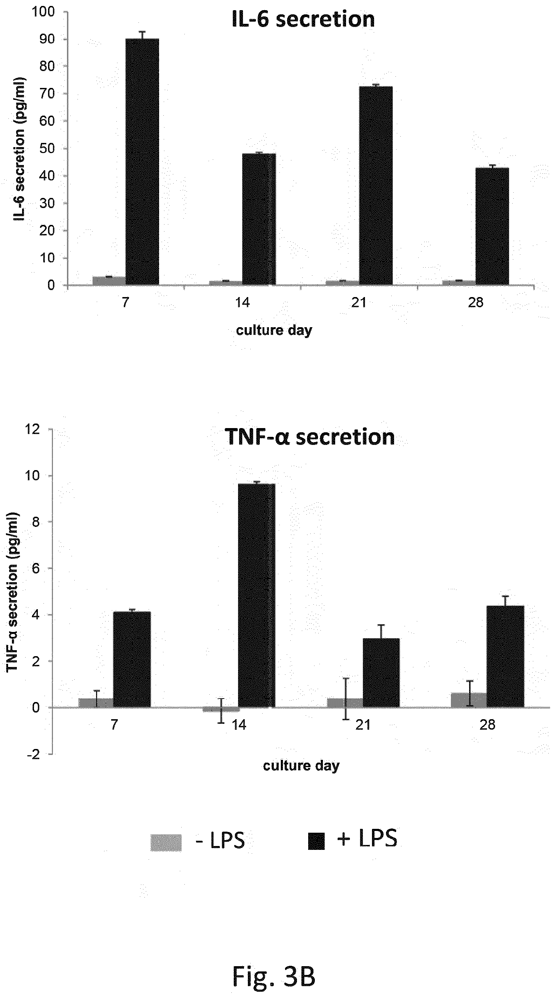

Fig. 3B: Induction of liver-disease as determined by secretion of inflammatory cytokines. Human Liver Microtissue co-cultures were treated with lipopolysaccharides for 48 hours once per week for a total of 4 weeks (28 days). The supernatants were analysed for IL-6 and TNF-α induction. Inflammation is thought to be the second step towards recapitulating NASH *in vitro*

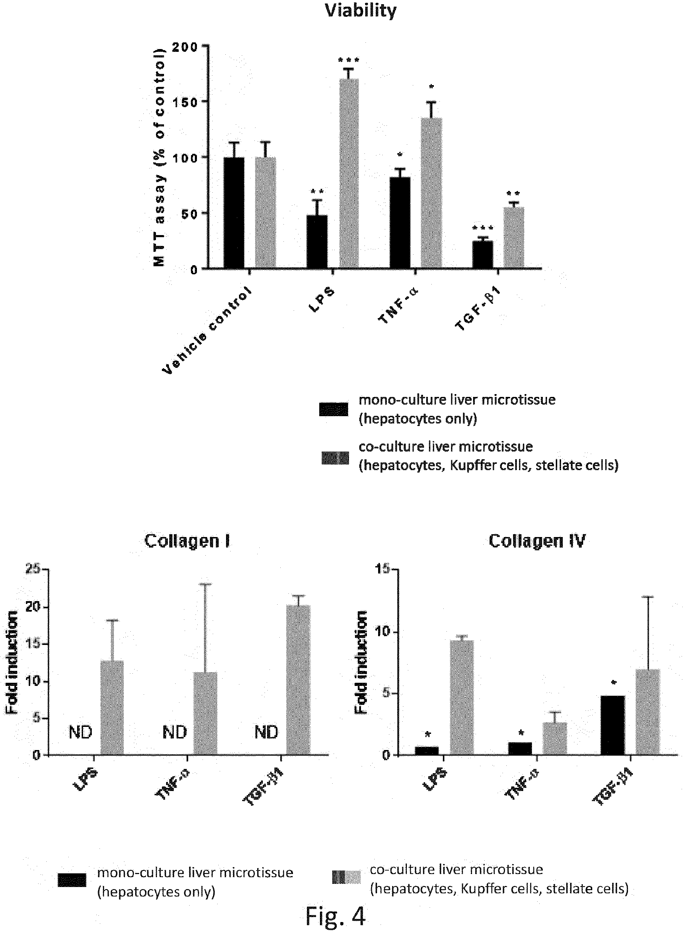

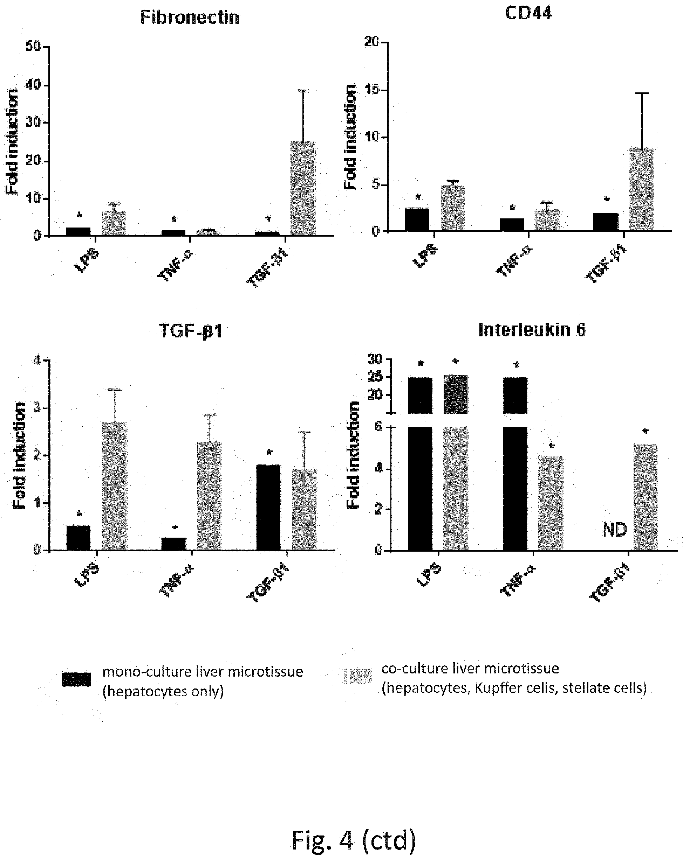

Fig. 4: MTT assay shows enhanced viability in liver cell triple-cultures as compared to hepatocyte mono-culture in presence of LPS and pro-inflammatory cytokines, TNF-α and TGF-β1 (Black bars: hepatocyte mono-culture. Grey bars: hepatocyte + inflammatory cells + stellate cells co-culture). Induction of fibrosis biomarkers, Collagen I and IV upon treatment with inflammation-stimuli LPS or TNF-α and pro-fibrotic TGF- β1 signaling molecule. Gene expression of fibrotic markers (Fibronectin, CD44) and cytokines (TGF-β1, IL-6) in human liver microtissues exposed to LPS, TNF-α and TGF-β1. mRNA was extracted using TRIzol conventional procedure and fold induction were calculated as 2^(-ΔΔCT) for each sample and vehicle control and expressed as mean fold induction ± S.E.M of three replicates with six MTs each. Actin was used as reference gene for each sample. ND: no-detected values. * ; P ≤ 0.05.

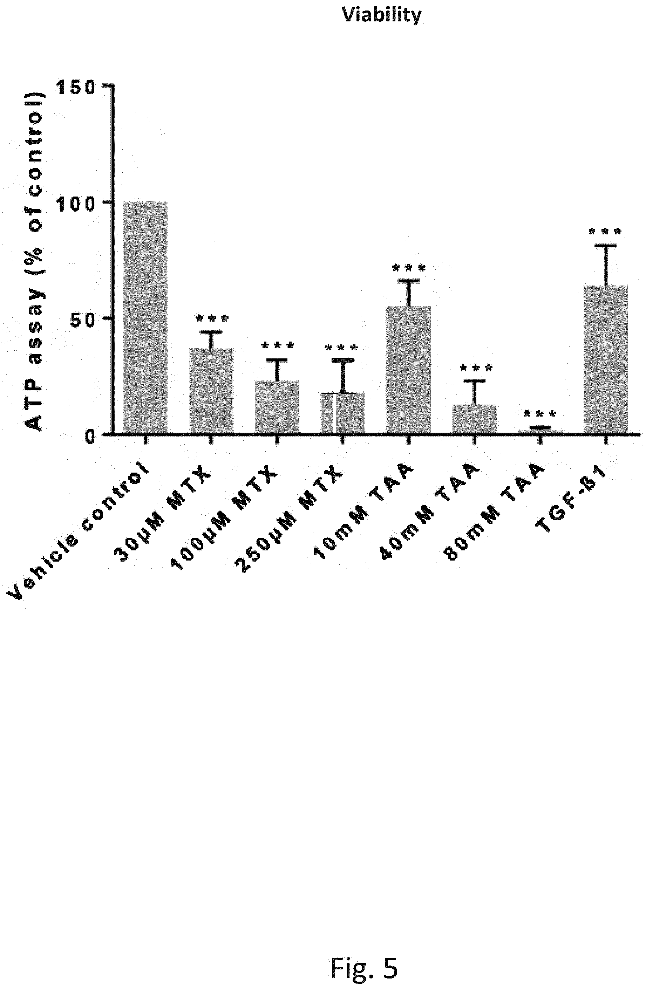

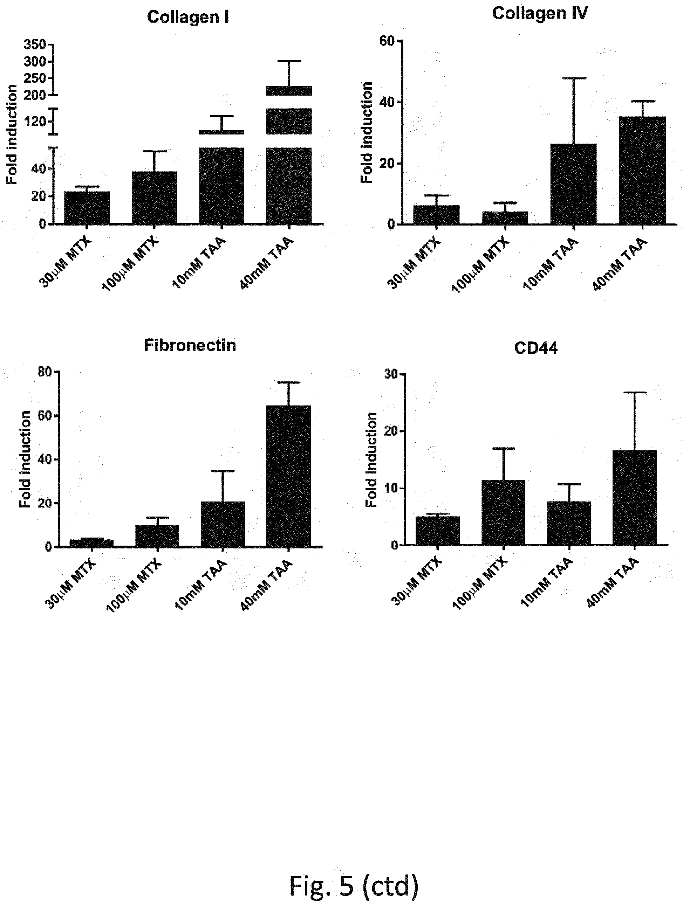

Fig. 5: Dose-dependent loss of viability or metabolic activity observed in liver MT triple-culture (hepatocyte + inflammatory cells + stellate cells) upon treatment with pro-fibrotic compounds MTX and TAA for 14 days, while pro-fibrotic gene expression (Collagen I, Collagen IV, CD44, Fibronectin) was greatly enhanced. MT mRNA was extracted using TRIzol conventional procedure and fold induction were calculated as 2^(-ΔΔCT) for each sample and negative control and expressed as mean fold induction ± S.E.M. of three replicates with six MTs each. Actin was used as reference gene for each sample.

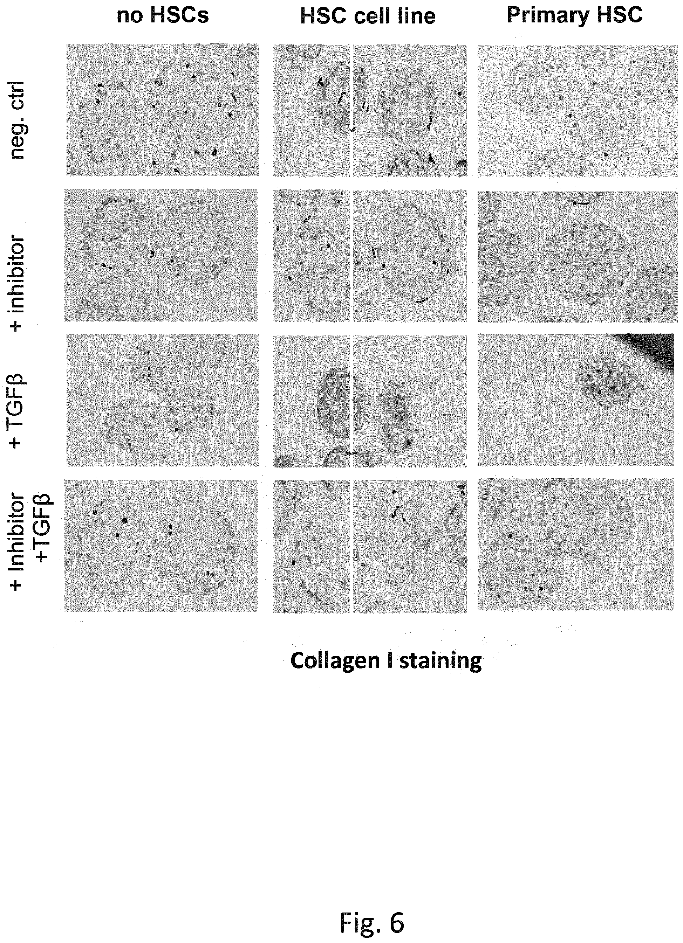

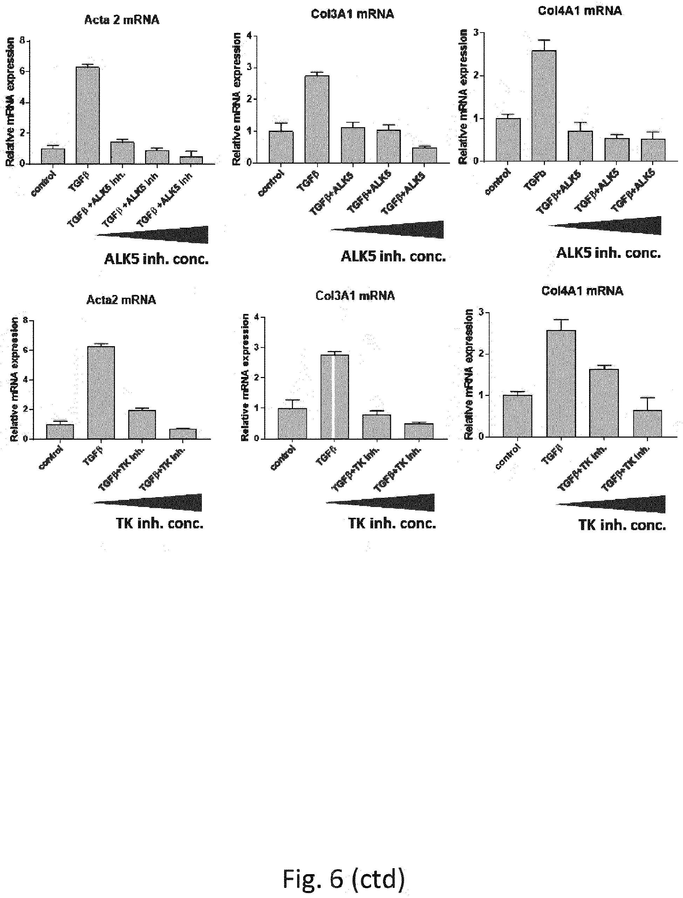

Fig. 6: Therapeutic effect of inhibition of pro-fibrotic effector pathways (TGF-βR, TK-R) was confirmed in triple co-culture liver MTs. Human liver MTs prepared form primary human hepatocytes, primary human Kupffer cells and primary human stellate cells where treated with TGF-β1 alone, or in combination with undisclosed inhibitors of i) TGF-β1 receptor, ii) ALK5, and iii) Tyrosine Kinase receptors. Progression of fibrosis by anti-fibrotic drug treatment in presence of pro-fibrotic agent TGF-β1 was clearly blunted as demonstrated by immunohistochemical staining of collagen I after 7 days of treatment. In another approach, the ALK5 inhibitor as well as the TK inhibitor showed a dose-dependent downregulation of the pro-fibrotic genes α-SMA (Acta 2), Collagen 3A1 and Collagen 4A1. In conclusion, these data demonstrate that the triple-culture model is suitable to assess anti-fibrotic properties of candidate compounds intended to be applied as prophylactic treatment.

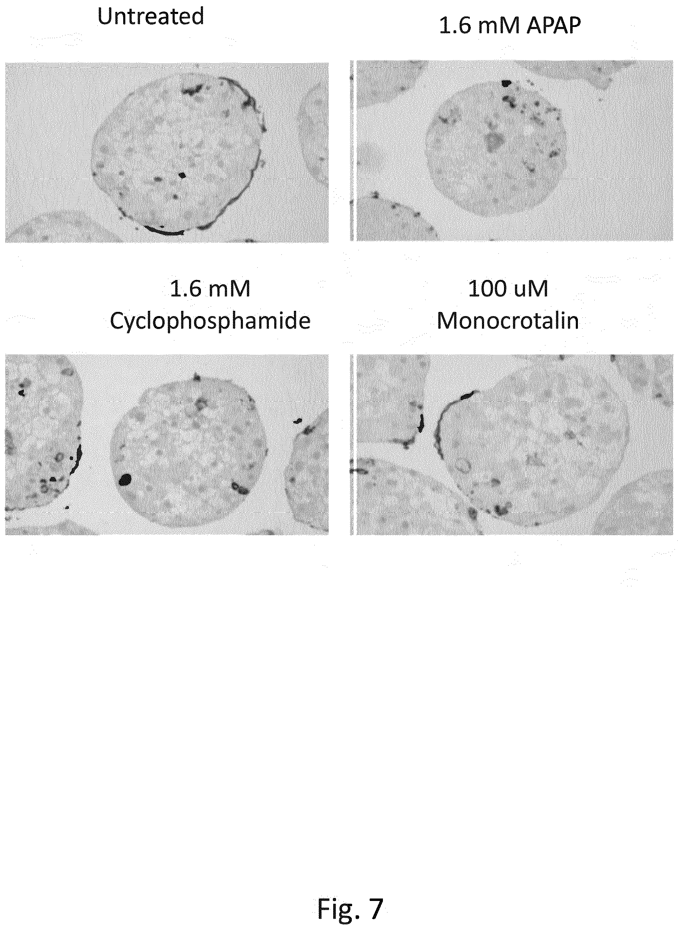

Fig. 7: Assessment of LEC-specific toxicity. LEC-coated microtissues were treated with Acetaminophen (APAP), Cyclosphosphamide and Monocrotaline for 5 days at the indicated concentrations, resulting in reduced LEC-presence on microtissues after treatment, as visualized by immunohistochemical staining for endothelial cell marker CD31.

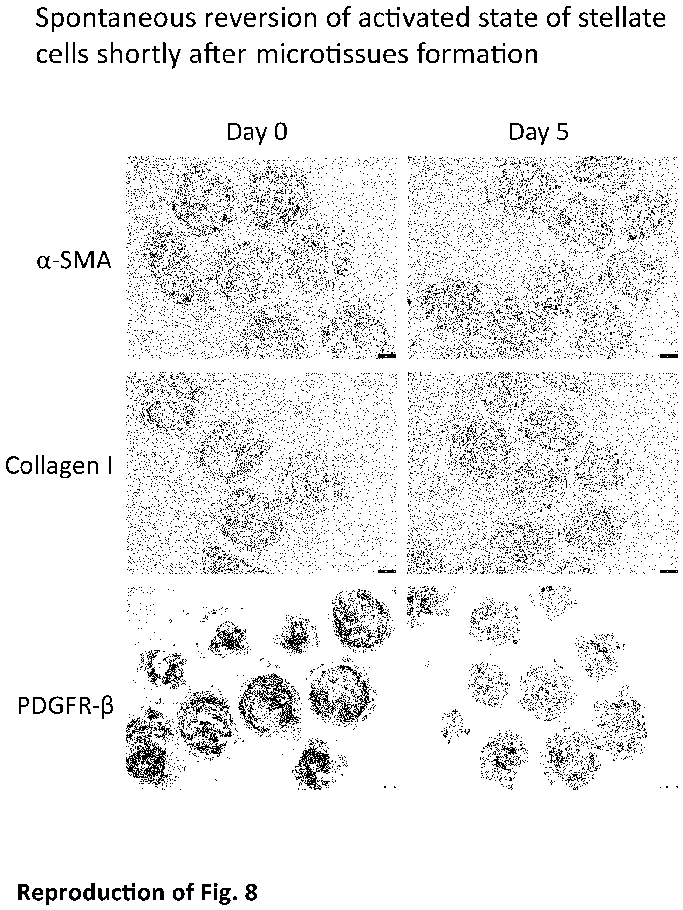

Fig. 8: Liver microtissues spontaneously revert to a more quiescent state after short term culture post production. Liver microtissues composed of primary hepatocytes, Kupffer cells and stellate cells where harvested 5 days after culture in specific maintenance medium and processed for immune histology. All three pro-fibrotic markers, α-SMA, Collagen I and PDGFR-β where less abundant than in MTs processed instantly after spheroid formation.

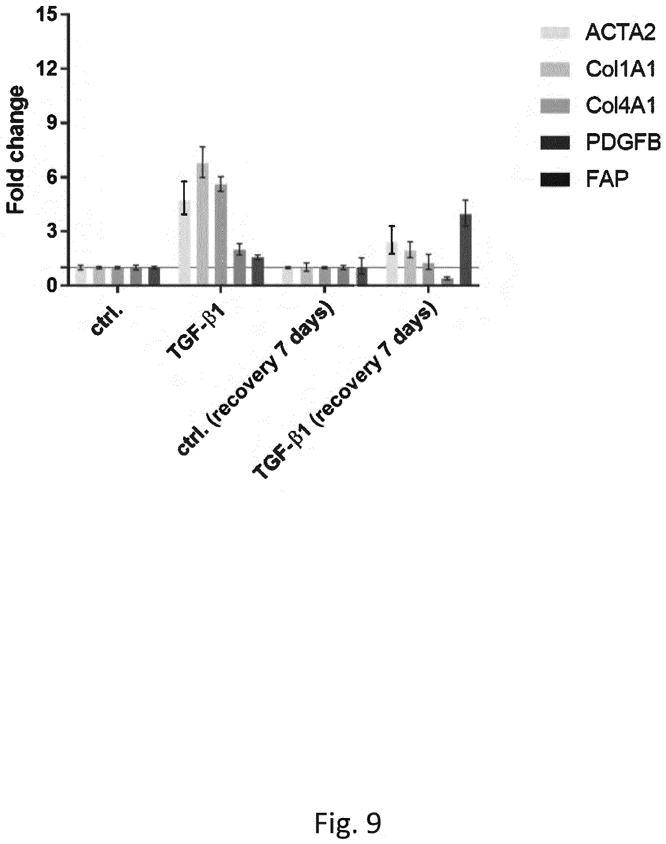

Fig. 9: Spontaneous reversion of fibrotic state. Liver microtissue composed of primary hepatocytes, Kupffer cells and stellate cells where treated with pro-fibrotic TGF-β1 for 7 days, upon wich MTs were washed and further incubated in specified maintenance medium in absence of pro-fibrotic agents for another 7 days. Gene expression profiling for fibrotic markers revealed reduced transcript levels after at the end of the recovery phase when normalized to respective untreated controls.

### Example 1: Production of triple cell type spheroidal microtissues (hepatocytes, Kupffer cells, stellate cells)

- After growth in 2D culture homo- and heterotypic microtissues were produced in hanging drops using InSphero's GravityPLUS^{™} (InSphero) platform. For protocols see WO2015158777A1
- First, hepatocytes and hepatic stellate cells were detached from cell culture flasks
- Therefore, medium was aspirated and the cell monolayer was washed with phosphate buffered saline (PBS)
- Detaching of hepatocytes and HSCs was by Trypsin treatent
- After optical confirmation of dissociation, thaw/wash medium was added, stopping the enzymatic reaction
- Kupffer cells were thawed and directly used for MT production
- Hepatocytes, HSCs and KCs were separately washed in thaw/wash medium by centrifugation
- After the centrifugation step, the cells were resuspended in thaw/wash medium
- Their cell number and viability was assessed
- For MT production cell suspensions of the desired composition were prepared in human liver maintenance medium.

### Example 2: Coating of spheroidal microtissues with liver endothelial cells (LEC)

- After artificial spheroidal microtissue formation (Day 4), LEC have been thawed, washed with LEC Coating Medium - and centrifuged
- In addition to the centrifugation step, the cells were resuspended in LEC Coating Medium and their cell number and viability has been assessed
- For LEC seeding and coating of the spheroidal microtissues a cell suspension of the desired composition was prepared in LEC Coating Medium
- spheroidal microtissues are being provided in suitable wells or vessel
- 5 µl of the LEC suspension are dispensed to each well or vessel.
- LEC sink down and get in contact with the microtissues and the extracellular matrix surrounding them
- After 2 days of incubation, microtissues are fully coated with LECs

### Example 3: Induction of steatotic phenotype by lipid loading of liver microtissues

- Dissolve FFA-free BSA at RT for 1h in H2O for desired stock solution concentration
- Dissolve desired amount of OA-Na in 1 mL 0.1 M NaOH --> at 70 °C
- Dissolve desired amount of PA-Na 1 mL 0.1 M NaOH --> at 70 °
- Keep the FFAs on 70°C, while the BSA is placed on 37°C.
- Mix FFA with BSA with a magnetic stirrer (for 30 min)
- Dilute BSA with 0.1 M NaOH -> vehicle control
- Cool down to RT, sterile filter, aliquot and freeze.
- For a final fatty acid (FA) solution dilute master stock in medium.
- Replace medium of liver microtissues with FA-containing medium and incubate 2-7 days.

Oleate (OA), a monounsaturated omega-9 fatty acid, incorporates in hepatocytes within small lipid droplets (microvesicular steatosis), while palmitic acid (PA), saturated hexadecanoic acid, shows a rather macrovesicular phenotype (Fig. 3A1). Lipid accumulation is the first step for recapitulating NASH (Non-Alcoholic SteatoHepatitis) in vitro. Indeed, macrovesicular steatosis has been associated with lobular inflammation and fibrosis (Mulder et al., 2015).

Lipid loading shows a dose dependent effect (Fig. 3A2). While increasing concentrations of OA demonstrate saturation regarding lipid droplet density, PA administration showed reduced lipid incorporation at concentrations higher than PA3. This observation is suggestive of reduced metabolic activity or even compromised viability of liver microtissues at higher PA concentrations. A combination of OA/PA at a molar ratio of 2:1 shows a mixed phenotype with both features of micro- and macrovesicular steatosis. ATP measurement confirms the morphological observations, with decreased values in microtissues exposed to PA4 and higher, particularly after long-term treatment (Fig. 3A3). PA was less harmful when combined with OA, as demonstrated by ATP levels comparable to control samples (BSA). These data confirm the general concept of saturated fatty acids being capable of progressing NAFLD to NASH (Leamy et al., 2013), most likely by inducing ER-stress response, ROS formation, release of pro-inflammatory cytokines and activation of inflammasomes. Hereby Kupffer cells play an important role in promoting the inflammatory response (Dixon et al., 2012) up to the point of releasing chemokines for attracting circulating monocytes to further progress into an inflammatory state, paralleled by activation of stellate cells.

### Example 4: Induction of inflammatory response and liver fibrosis

In order to assess the responsiveness towards prototypic inflammatory stimuli and pro-fibrotic stimuli on a gene-expression level, mono-cultured (HepaRG) and triple/co-cultured (HepaRG THP1/hTERT-HSC) liver MT were stimulated with inflammatory inducers (LPS or TNF-α) and the pro-fibrotic cytokine TGF-β1 to assess the cellular responses. Significant differences in the responses of the two types of MT were observed (Fig. 4). The treatment with LPS and TNF-α resulted in increased cellular viability of co-cultured microtissues, whereas TGF-β1 decreased viability after 14 days of treatment. Analysis of gene expression changes after treatments showed a strong induction of the pro-fibrotic markers Collagen I and IV, fibronectin, CD44 and TGF-β1 in co-cultured MTs, indicating presence of extracellular matrix protein transcription and further stimulation of pro-fibrotic conditions. HepaRG monocultures, however, exhibited only moderate or no response towards pro-fibrotic stimuli. For example, mRNA of collagen I was only detected in the co-culture system, but not in the monocultures. Interestingly, monocultures were strongly responsive towards LPS and TNF-α for IL-6 gene induction, demonstrating the responsiveness for inflammatory stimuli of these hepatocyte-like cells in spheroidal microtissues. TGF-β1 did not induce IL-6 gene expression in monocultures, but was observed in co-cultures, suggesting monocytes as the predominant source of IL-6 (Fig. 4).

### Example 5: A method to which employs the triple/quadruple liver microtissue model to assess adverse liver effects such as fibrosis and cirrhosis

In order to test whether the described in vitro model is capable of recapitulating induction of liver fibrosis upon treatment with clinically relevant compounds, we tested the pro-fibrotic chemicals methotrexate (MTX) and Thiocetamide (TAA), which were both shown to cause hepatic fibrosis in vivo. Exposure of co-cultured Microtissues to MTX and TAA over 14 days showed a dose-dependent decrease of MT viability with both MTX and TAA (Fig. 5). Gene expression analysis showed reproducible, also dose-dependent transcriptional induction of collagen I, collagen IV, fibronectin I and CD44 (Fig. 5).

### Example 6: A method to assess adverse effects on liver endothelial cells

Spheroidal liver microtissues containing hepatocytes and Kupffer cells were coated with liver endothelial cells (LEC) and subjected to 5 days treatment with sub-toxic doses of Acetaminophen (APAP), Cyclophosphamide and Monocrotaline (Fig. 7). After the treatment, microtissues were collected, formalin fixed and paraffin embedded, sliced and stained by immunohistochemistry for the LEC-marker CD31. The marker was present in the untreated control, confirming the coating of the LEC of the spheroidal Liver Microtissue. After treatment with the LEC-toxicants acetaminophen, cyclosphosphamide and monocrotalin, however, the CD31-marker protein was heavily reduced or completely absent. This suggests that these compounds indeed are toxic to LECs at the tested concentration.

### Example 7: Inhibition of fibrosis progression (Fig.6)

Human liver microtissues (+Kupffer cells) without stellate cells and with either a stellate cell line or primary human stellate cells were treated with a fibrosis inducer (TGF-β1) and in combination with an undisclosed fibrosis inhibitor. Histological analysis after 7 days treatment demonstrates significant reduction of basal fibrosis represented by diminished staining for collagen I in cell line derived HSC cultures compared to the untreated control. Even more pronounced is the difference between TGF-β1 treated stellate cell comprising microtissues and TGF-β1/inhibitor treated cultures. While TGF-β1 treated microtissues display a severe fibrotic phenotype with collagen deposition, co-treatment nearly completely inhibited the fibrotic phenotype. When co-treated with TGF-β1, the anti-fibrotic properties of another two compounds acting on different targets involved in the progression of fibrosis, i.e. an ALK5 inhibitor or a Tyrosine Kinase (TK) inhibitor, could also be demonstrated on the basis of the blunted induction of transcripts of the fibrotic markers α-SMA, Collagen 3A1 and Collagen 4A1. This again demonstrates the prevention of the fibrotic phenotype by pharmacological intervention and thus the applicability of 3-cell type liver microtissues as a model for testing anti-fibrotic properties of putative therapeutic compounds.

### Example 8: Spontaneous reversion of stellate cell activation or fibrotic phenotype

Stellate cells do show explicit dynamic behavior acting in response to tissue injury (e.g. trauma, viral infection, inflammatory processes, or ischemia) by transdifferentiating from a lipocyte-like cell into a contractile and highly proliferative myofibroblast-like cell which produce a variety of extracellular matrix components as part of the scar-formation process. This very well reflects one of their primary biological functions, namely the initiation of tissue repair upon various types of tissue insult. Therefore stellate cells are very sensitive to environmental alterations beyond normal physiological limits. This also is manifested under in vitro conditions usually applied during the cell expansion phase, namely culturing stellate cells on conventional cell culture plastic dishes. This by far does not represent their natural environment which consequently leads to a change in their phenotype towards an activated state reflecting the repair mode similar but not identical (see De Mincis et al., 2007) to in vivo conditions after tissue injury. Stellate cells revert to their normal quiescent state once tissue injury has been resolved (Kisseleva et al., 2012). This exact dynamic behavior is also observed in the course of in vitro expansion and subsequent 3D co-culture in combination with hepatocytes and Kupffer cells, where stellates undergo temporary activation manifested by elevated pro-fibrotic markers (e.g. α-SMA, Collagen I, PDGFR-β). Reversion can be achieved within a few days after set-up of 3D culture simply by co-culture with hepatocytes and Kupffer cells in 3D-configuration in the absence of any stressor signal, i.e. in regular culture conditions (37°C, 5% CO₂, in maintenance medium optimized for liver 3D culture), as illustrated in Fig. 8. This is the first time such spontaneous reversion from an activated state of stellate cells to a more quiescent state phenotype is observed in vitro not requiring particular treatment with medium additives (growth factors, such as FGF2, EGF, retinol and free fatty acids) as recently described by Taghdouini et al., (2015). As this spontaneous type of reversion was not observed in in 2D culture set-up or 3D mono-culture involving only stellate cells, it strongly suggests that only the interplay of stellate cells with other livers cells, mainly hepatocytes and Kupffer cells, fostered by the 3D spheroid configuration allows this dynamic, bi-phasic behavior of this cell type.

The strength of the proposed liver in vitro culture format for the study of the dynamic behavior upon pharmacological, inflammatory or dietary stressors in the progression of fatty liver disease has also been demonstrated in the case of treatment with the pro-fibrotic cytokine TGF-β1. Gene expression levels of fibrotic markers (ACTA2, Col1A1, Col4A1, PDGFB) were clearly upregulated after stimulation with TGF-β1, but reversed to almost baseline levels within 7 days after TGF-β1 treatment was abolished (Fig. 9). This observation is indicative of the capacity of the model system to revert back from a pathophysiological to a normal physiological state by resolving the fibrotic phenotype, a fundamental prerequisite for in vitro test-system to be applied for testing therapeutic strategies after the onset of the disease. Interestingly aside from declined gene expression levels of the fibrotic markers after the wash-out phase, the simultaneous upregulation of the fibroblast activation protein (FAP) confirms the reversion from the diseased into a more healthy state. FAP has recently been described to be involved in the clearance in collagen catabolism and clearance is believed to be important for resolving scar after tissue injury and remodeling (Fan et. al., 2016).

### Example 9: Metabolic liver disease testing platform (Fig. 1A, 1B)

Based on the described experiments and related observations an *in vitro* platform is proposed, which is capable to allow following investigative modes:
1. Study of disease progression and testing of anti-NASH/Fibrosis therapeutic or preventive strategies by pharmacological and/or dietary interventions, starting from different entry points:
   o Liver model with pro-activated stellate cells (T₁, right after tissue formation, process [a], described in example 1)
   o Liver model at basal state, after reversion from activated post-production state (process [b], described in example 8), or further progressed to either of the following states:
      ▪ Liver model with insulin resistant state (process [b']) introduced by
         - High glucose/fructose
         - High insulin
         - High free fatty acids (SFA>MUFA>PUFA)
         - Adipocyte derived cytokines TNF-α, IL-1β, and IL-6
         - Resistin
         - Glucocorticoids
         - or a combination of above
      ▪ Liver model with steatotic state, (process [c], described in example 3). • MUFA:SFA molar ratio ≥1
      ▪ Liver model with inflammatory state, (process [d], described in example 4).
      ▪ Liver model with fibrotic state, (process [e], described in example 4 and 5).
      ▪ Liver model with NASH/Fibrosis phenotype (option 1), (by sequential or simultaneous application of process [b'], [c], [d], and [e]).
      ▪ Liver model with NASH/Fibrosis phenotype (option 2), by treatment with pro-inflammatory dietary free fatty acid composition (process [f])
         - SFA only
         - or MUFA:SFA molar ratio <1
      Most common SFAs in human diet are: Stearic acid, Palmitic acid, Myristic acid, Lauric acid, Capric acid, Caprylic acid, Caproic acid Most common MUFAs in human diet are: Oleic acid, Palmitoleic acid
   o Liver model at basal state or any of the above disease models in co-culture with either type of inflammatory cells
      - NK, NKT or iNKT cells,
      - T lymphocytes,
      - polymorphonuclear cells
      - CD8 lymphocytes
      - Peripheral Blood Mononuclear Cells (PBMCs)
      - any of above inflammatory cells possibly haplotype-matched to liver cells used for tissue formation

      ▪ as static co-culture or in a microfluidic device allowing free floating of inflammatory cells with immobilized liver microtissues
2. Liver model at basal state or any of the above models representing advanced states of diseased liver for safety assessment of drug candidates with suspected liver toxic liabilities, as described in example 5 and 6.

The versatility of the platform allows different modes of implementation or commercialization:
2. Provision of aforementioned basal liver models and disease models as assay ready system, including
   a. Basal maintenance medium
   b. Diseased state-specific media
   c. QC certification
   d. Reference test compounds
3. Provision of aforementioned basal liver models and supplements for the induction of any of above mentioned disease states as assay-ready system, including
   a. Basal maintenance medium
   b. QC certification
   c. Reference test compounds

### References

- van Grunsven L.A., Advanced Drug Delivery Reviews. 2017; 121 pp: 133-146
- Kelm et al., J Biotechnol. 2005; 118 pp: 213-29
- Leite S. et al., Biomaterials. 2016 ; 78 pp: 1-10
- Liedtke C et al., Fibrogenesis Tissue Repair. 2013; 6: 19
- Ming-Hui Fan et. al., J Biol Chem. 2016; 291(15): 8070-8089
- Puche, Saiman, & Friedman, Compr Physiol. 2013; 3(4):1473-92
- Taghdouini et al., Fibrogenesis Tissue Repair. 2015 ; 8:14
- Nguyen DG et al., PLoS ONE 11(7) (2016): e0158674
- The Importance of Multicellularity in Liver Homeostasis and Injury, 2015, Organovo
- Duarte et al., BioMed Research International Volume 2015 (2015), Article ID 984578
- Mulder et al., Inflamm Cell Signal 2015; 2: e804
- Leamy et al., Prog Lipid Res. 2013 Jan; 52(1): 165-174
- Dixon et al., Lab Invest. 2012 May ; 92(5): 713-723
- Fan et. al., J Biol Chem. 2016; 291(15): 8070-8089
- Taghdouini et al., Fibrogenesis Tissue Repair. 2015;8:14
- Kisseleva et al., Proc Natl Acad Sci. 2012;109(24): 9448-53
- DeMincis et al., Gastroenterology. 2007 May;132(5):1937-46

## Claims

1. A method of providing a model platform for liver disease modelling, the method comprising preparing an artificial spheroidal microtissue comprising primary human hepatocytes, primary human hepatic stellate cells and primary human Kupffer cells, comprising
(i) co-culturing primary human hepatocytes, primary human Kupffer and primary human stellate cells in a 3D hanging drop system or a 3D low adherence well in maintenance medium for liver 3D culture without any of a growth factor, retinol and free fatty acid supplementation, for 5 to 7 days, such that the artificial spheroidal microtissue adopts a basal state, wherein the stellate cells have a quiescent state, and
(ii) subsequently incubating the microtissue for 1 to 14 days in the presence of a monounsaturated fatty acid (MUFA) and a saturated fatty acid (SFA) in a molar ratio of MUFA:SFA of ≥ 1, thereby inducing a steatotic state in the microtissue,
wherein the microtissue is grown and cultivated in a 3D hanging drop system or a 3D low adherence well.

2. The method according to claim 1, wherein the MUFA is oleic acid or palmitoleic acid, and the SFA is selected from the group consisting of stearic acid, palmitic acid, myristic acid, lauric acid, capric acid, caprylic acid, and caproic acid.

3. The method according to claim 2, wherein the molar ratio of oleate (OA) to palmitic acid (PA) is 2:1.

4. The method according to any of claims 1 to 3, wherein the steatotic state are of a mixed phenotype **characterized by** micro- and macrovesicular steatosis.

5. The method according to any of claims 1 to 4, further comprising stimulating the microtissue with the inflammatory inducers LPS or TNF-α or the pro-fibrotic cytokine TGF-β1 for 1 to 7 days, thereby inducing an inflammatory state in the microtissue.

6. The method according to claim 5, further comprising stimulating the microtissue with the pro-fibrotic chemicals methotrexate (MTX) or Thiocetamide (TAA) for 1 to 7 days or 14 days, thereby inducing a fibrotic state in the microtissue.

7. The method according to any of claims 1 to 6, wherein the microtissue prior to incubating in the presence of a MUFA and a SFA
a) has a diameter of between ≥ 30 µm and ≤ 500 µm, or
b) comprises between ≥ 500 and ≤ 10000 cells in total.

8. A method of preparing a spheroidal microtissue comprising at least hepatocytes, hepatic stellate cells, hepatic inflammatory cells, and further endothelial cells, the method comprising the steps of:
(a) producing or providing a microtissue comprising primary human hepatocytes, human Kupffer cells and human hepatic stellate cells by co-culturing primary human hepatocytes, primary human Kupffer and primary human stellate cells in a 3D hanging drop system or a 3D low adherence well in maintenance medium for liver 3D culture without any of a growth factor, retinol and free fatty acid supplementation, for 5 to 7 days, such that the artificial spheroidal microtissue adopts a basal state, wherein the stellate cells have a quiescent state,
(b) producing or providing a suspension comprising sinusoidal endothelial cells,
(c) dispensing said suspension into wells or vessels comprising one or more microtissues of step (a), and
(d) incubating said wells or vessels to let sinusoidal endothelial cells adhere to the microtissues.

9. The method according to claim 8, wherein the sinusoidal endothelial cells form a cell layer around the three dimensional microtissue comprising at the remaining cell types.

10. The method according to any of the preceding claims, wherein the microtissue prior to incubating in the presence of a MUFA and a SFA shows reduced abundancy of at least one of profibrotic marker selected from the group consisting of α-SMA, Collagen I and PDGFR-β than a microtissue formed instantly after spheroid formation, without intermediate culture.

11. A method for testing compounds suspected of promoting, causing, or increasing the risk of NAFLD/NASH/Fibrosis,
the method comprising co-culturing for 5 to 7 days primary human hepatocytes, primary human Kupffer and primary human hepatic stellate cells in a 3D hanging drop system or a 3D low adherence well in maintenance medium for liver 3D culture without any of a growth factor, retinol and free fatty acid supplementation, such that an artificial spheroidal microtissue is obtained that adopts a basal state, wherein the stellate cells have a quiescent state,
the method further comprising the steps of
a) exposing the obtained microtissue to one or more test compounds suspected of promoting, causing, or increasing the risk of NAFLD/NASH/Fibrosis,
b) carrying out an endpoint analysis of at least one genetic, physiologic and/or morphologic parameter relevant to the liver pathology of NAFLD/NASH/Fibrosis,
wherein the microtissue is grown and cultivated in a 3D hanging drop system or a 3D low adherence well.

12. The method of claim 11, wherein the endpoint analysis refers to at least one parameter selected from:
• Release of one or more biomarkers
• Lipid content of the microtissue cells
• Histology of the microtissue and its cells (disrupted/non disrupted)
• RNA expression marker such as alpha-smooth muscle actin, COL1A1, Vimentin or TIMP-1
• Size or volume of the microtissue, and/or
• Mitochondrial activity.

## Patentansprüche

1. Verfahren zum Bereitstellen einer Modellplattform für die Modellierung von Lebererkrankungen, wobei das Verfahren die Herstellung eines künstlichen sphäroidalen Mikrogewebes aufweist, das primäre menschliche Hepatozyten, primäre menschliche Lebersternzellen und primäre menschliche Kupffer-Zellen aufweist, aufweisend
(i) primäre menschliche Hepatozyten, primäre menschliche Kupffer-Zellen und primäre menschliche Sternzellen in einem 3D-Hängetropfensystem oder in einer 3D-Vertiefungsplatte mit geringer Adhäsion in einem Erhaltungsmedium für 3D-Leberkultur ohne Zugabe von Wachstumsfaktoren, Retinol und freien Fettsäuren für 5 bis 7 Tage co-kultivieren, so dass das künstliche sphäroidale Mikrogewebe einen Basalzustand annimmt, in dem sich die Sternzellen in einem Ruhezustand befinden, und
(ii) anschließend das Mikrogewebe für 1 bis 14 Tage in Gegenwart einer einfach ungesättigten Fettsäure (engl. monounsaturated fatty acid, MUFA) und einer gesättigten Fettsäure (engl. saturated fatty acid, SFA) in einem Molverhältnis von MUFA:SFA von ≥ 1 inkubieren, wodurch ein steatotischer Zustand im Mikrogewebe induziert wird,
wobei das Mikrogewebe in einem 3D-Hängetropfensystem oder in einer 3D-Vertiefungsplatte mit geringer Adhäsion gezüchtet und kultiviert wird.

2. Verfahren nach Anspruch 1, wobei die MUFA Ölsäure oder Palmitoleinsäure ist und die SFA aus der Gruppe ausgewählt ist, die aus Stearinsäure, Palmitinsäure, Myristinsäure, Laurinsäure, Caprinsäure, Caprylsäure und Capronsäure besteht.

3. Verfahren nach Anspruch 2, wobei das Molverhältnis von Oleat (OA) zu Palmitinsäure (PA) 2:1 beträgt.

4. Verfahren nach einem der Ansprüche 1 bis 3, wobei der steatotische Zustand einen gemischten Phänotyp aufweist, der durch mikro- und makrovesikuläre Steatose gekennzeichnet ist.

5. Verfahren nach einem der Ansprüche 1 bis 4, wobei das Verfahren weiterhin aufweist, das Mikrogewebe für 1 bis 7 Tage mit den Entzündungsinduktoren LPS oder TNF-α oder dem profibrotischen Cytokin TGF-β1 zu stimulieren, wodurch ein Entzündungszustand im Mikrogewebe induziert wird.

6. Verfahren nach Anspruch 5, das weiterhin aufweist, das Mikrogewebe mit den profibrotischen Chemikalien Methotrexat (MTX) oder Thiocetamid (TAA) für 1 bis 7 Tage oder 14 Tage zu stimulieren, wodurch ein fibrotischer Zustand im Mikrogewebe induziert wird.

7. Verfahren nach einem der Ansprüche 1 bis 6, wobei das Mikrogewebe vor dem Inkubieren in Gegenwart einer MUFA und einer SFA
a) einen Durchmesser zwischen ≥ 30 µm und ≤ 500 µm hat oder
b) insgesamt zwischen ≥ 500 und ≤ 10000 Zellen umfasst.

8. Verfahren zur Herstellung eines sphäroidalen Mikrogewebes, das mindestens Hepatozyten, hepatische Sternzellen, hepatische Entzündungszellen und weitere Endothelzellen aufweist, wobei das Verfahren die folgenden Schritte aufweist:
(a) ein Mikrogewebe, das primäre menschliche Hepatozyten, menschliche Kupffer-Zellen und menschliche hepatische Sternzellen aufweist, durch Co-kultivieren von primären menschlichen Hepatozyten, primären menschlichen Kupffer-Zellen und primären menschlichen Sternzellen in einem 3D-Hängetropfensystem oder einer 3D-Vertiefungsplatte mit geringer Adhäsion in einem Erhaltungsmedium für 3D-Leberkultur ohne Zugabe von Wachstumsfaktoren, Retinol und freien Fettsäuren herstellen oder bereitstellen, so dass das künstliche sphäroidale Mikrogewebe einen Basalzustand annimmt, in dem sich die Sternzellen in einem Ruhezustand befinden,
(b) eine Suspension herstellen oder bereitstellen, die sinusoidale Endothelzellen aufweist,
(c) die Suspension in Vertiefungen oder Gefäßen dispergieren, die ein oder mehrere Mikrogewebe aus Schritt (a) aufweisen, und
(d) die Vertiefungen oder Gefäße inkubieren, damit die sinusoidalen Endothelzellen an den Mikrogeweben anhaften.

9. Verfahren nach Anspruch 8, wobei die sinusoidalen Endothelzellen eine Zellschicht um das dreidimensionale Mikrogewebe bilden, das die übrigen Zelltypen aufweist.

10. Verfahren nach einem der vorangehenden Ansprüche, wobei das Mikrogewebe vor der Inkubation in Gegenwart einer MUFA und einer SFA eine verringerte Fülle an mindestens einem profibrotischen Marker, ausgewählt aus der Gruppe bestehend aus α-SMA, Kollagen I und PDGFR-β, aufweist als ein Mikrogewebe, das unmittelbar nach der Sphäroidbildung ohne Zwischenkultur gebildet wurde.

11. Verfahren zum Testen von Verbindungen, die im Verdacht stehen, NAFLD/NASH/Fibrose zu fördern, zu verursachen das Risiko für NAFLD/NASH/Fibrose zu fördern, zu verursachen oder das Risiko dafür zu erhöhen,
wobei das Verfahren aufweist, für 5 bis 7 Tage primäre menschliche Hepatozyten, primäre menschliche Kupffer-Zellen und primäre menschliche Lebersternzellen in einem 3D-Hängetropfensystem oder einer 3D-Vertiefungsplatte mit geringer Adhäsion in einem Erhaltungsmedium für 3D-Leberkultur ohne Zugabe von Wachstumsfaktoren, Retinol und freien Fettsäuren zu co-kultivieren, so dass ein künstliches sphäroidales Mikrogewebe erhalten wird, das einen Basalzustand annimmt, in dem sich die Sternzellen in einem Ruhezustand befinden,
wobei das Verfahren weiterhin die folgenden Schritte aufweist
a) das erhaltene Mikrogewebe einer oder mehreren Testverbindungen aussetzen, die im Verdacht stehen, NAFLD/NASH/Fibrose zu fördern, zu verursachen das Risiko für NAFLD/NASH/Fibrose zu fördern,
b) eine Endpunktanalyse mindestens eines genetischen, physiologischen und/oder morphologischen Parameters durchführen, der für die Leberpathologie von NAFLD/NASH/Fibrose relevant ist,
wobei das Mikrogewebe in einem 3D-Hängetropfensystem oder einer 3D-Vertiefungsplatte mit geringer Adhäsion gezüchtet und kultiviert wird.

12. Verfahren nach Anspruch 11, wobei sich die Endpunktanalyse auf mindestens einen Parameter bezieht, der ausgewählt ist aus:
• Freisetzung eines oder mehrerer Biomarker
• Lipidgehalt der Mikrogewebezellen
• Histologie des Mikrogewebes und seiner Zellen (zerstört/nicht zerstört)
• RNA-Expressionsmarker wie Alpha-Aktin der glatten Muskulatur, COL1A1, Vimentin oder TIMP-1
• Größe oder Volumen des Mikrogewebes und/oder
• Mitochondriale Aktivität.

## Revendications

1. Procédé de fourniture d'une plateforme modèle pour la modélisation de maladies hépatiques, le procédé comprenant la préparation d'un microtissu sphéroïdal artificiel comprenant des hépatocytes humains primaires, des cellules étoilées hépatiques humaines primaires et des cellules de Kupffer humaines primaires, comprenant :
(i) la culture conjointe d'hépatocytes humains primaires, de cellules de Kupffer humaines primaires et de cellules étoilées humaines primaires dans un système de gouttes suspendues en 3D ou dans un puits à faible adhérence en 3D dans un milieu de maintien pour culture hépatique 3D sans aucune supplémentation en facteur de croissance, rétinol et acide gras libre, pendant 5 à 7 jours, de sorte que le microtissu sphéroïdal artificiel adopte un état basal, dans lequel les cellules étoilées ont un état quiescent, et
(ii) l'incubation subséquente du microtissu pendant 1 à 14 jours en présence d'un acide gras mono-insaturé (MUFA) et d'un acide gras saturé (SFA) dans un rapport molaire MUFA:SFA ≥ 1, induisant de ce fait un état stéatosique dans le microtissu,
dans lequel le microtissu est cultivé et maintenu dans un système de gouttes suspendues en 3D ou dans un puits à faible adhérence en 3D.

2. Procédé selon la revendication 1, dans lequel le MUFA est de l'acide oléique ou de l'acide palmitoléique, et le SFA est sélectionné dans le groupe constitué de l'acide stéarique, l'acide palmitique, l'acide myristique, l'acide laurique, l'acide caprique, l'acide caprylique, et l'acide caproïque.

3. Procédé selon la revendication 2, dans lequel le rapport molaire d'oléate (OA) sur l'acide palmitique (PA) est de 2:1.

4. Procédé selon l'une quelconque des revendications 1 à 3, dans lequel l'état stéatosique est d'un phénotype mixte **caractérisé par** une stéatose micro- et macrovésiculaire.

5. Procédé selon l'une quelconque des revendications 1 à 4, comprenant en outre la stimulation du microtissu avec les inducteurs inflammatoires LPS ou TNF-α ou la cytokine pro-fibrotique TGF-β1 pendant 1 à 7 jours, induisant de ce fait un état inflammatoire dans le microtissu.

6. Procédé selon la revendication 5, comprenant en outre la stimulation du microtissu avec les produits chimiques pro-fibrotiques méthotrexate (MTX) ou thioacétamide (TAA) pendant 1 à 7 jours ou 14 jours, induisant de ce fait un état fibrotique dans le microtissu.

7. Procédé selon l'une quelconque des revendications 1 à 6, dans lequel le microtissu avant incubation en présence d'un MUFA et d'un SFA :
a) a un diamètre compris entre ≥ 30 µm et ≤ 500 µm, ou
b) comprend entre ≥ 500 et ≤ 10 000 cellules au total.

8. Procédé de préparation d'un microtissu sphéroïdal comprenant au moins des hépatocytes, des cellules étoilées hépatiques, des cellules inflammatoires hépatiques, et en outre des cellules endothéliales, le procédé comprenant les étapes suivantes :
(a) production ou fourniture d'un microtissu comprenant des hépatocytes humains primaires, des cellules de Kupffer humaines et des cellules étoilées hépatiques humaines par culture conjointe d'hépatocytes humains primaires, de cellules de Kupffer humaines primaires et de cellules étoilées humaines primaires dans un système de gouttes suspendues en 3D ou dans un puits à faible adhérence en 3D dans un milieu de maintien pour culture hépatique 3D sans aucune supplémentation en facteur de croissance, rétinol et acide gras libre, pendant 5 à 7 jours, de sorte que le microtissu sphéroïdal artificiel adopte un état basal, dans lequel les cellules étoilées ont un état quiescent,
(b) production ou fourniture d'une suspension comprenant des cellules endothéliales sinusoïdales,
(c) distribution de ladite suspension dans des puits ou récipients comprenant un ou plusieurs microtissus de l'étape (a), et
(d) incubation desdits puits ou récipients pour laisser les cellules endothéliales sinusoïdales adhérer aux microtissus.

9. Procédé selon la revendication 8, dans lequel les cellules endothéliales sinusoïdales forment une couche cellulaire autour du microtissu tridimensionnel comprenant les types cellulaires restants.

10. Procédé selon l'une quelconque des revendications précédentes, dans lequel le microtissu avant incubation en présence d'un MUFA et d'un SFA montre une abondance réduite d'au moins un marqueur profibrotique sélectionné dans le groupe constitué de α-SMA, Collagène I et PDGFR-β par rapport à un microtissu formé instantanément après la formation de sphéroïde, sans culture intermédiaire.

11. Procédé pour tester des composés suspectés de promouvoir, causer, ou augmenter le risque de NAFLD/NASH/Fibrose,
le procédé comprenant la culture conjointe pendant 5 à 7 jours d'hépatocytes humains primaires, de cellules de Kupffer humaines primaires et de cellules étoilées hépatiques humaines primaires dans un système de gouttes suspendues en 3D ou dans un puits à faible adhérence en 3D dans un milieu de maintien pour culture hépatique 3D sans aucune supplémentation en facteur de croissance, rétinol et acide gras libre, de sorte qu'un microtissu sphéroïdal artificiel soit obtenu qui adopte un état basal, dans lequel les cellules étoilées ont un état quiescent,
le procédé comprenant en outre les étapes suivantes :
a) exposition du microtissu obtenu à un ou plusieurs composés d'essai suspectés de promouvoir, causer, ou augmenter le risque de NAFLD/NASH/Fibrose,
b) réalisation d'une analyse de point final d'au moins un paramètre génétique, physiologique et/ou morphologique pertinent pour la pathologie hépatique de NAFLD/NASH/Fibrose,
dans lequel le microtissu est cultivé et maintenu dans un système de gouttes suspendues en 3D ou dans un puits à faible adhérence en 3D.

12. Procédé la revendication 11, dans lequel l'analyse de point final se réfère à au moins un paramètre sélectionné parmi :
• Libération d'un ou plusieurs biomarqueurs
• Contenu lipidique des cellules du microtissu
• Histologie du microtissu et de ses cellules (perturbé/non perturbé)
• Marqueur d'expression ARN tel que l'actine du muscle lisse alpha, COL1A1, Vimentine ou TIMP-1
• Taille ou volume du microtissu, et/ou
• Activité mitochondriale.
